# EUROPEAN PATENT APPLICATION

(11) **EP 3 726 535 A1**
(43) Date of publication of application: **21.10.2020**
(21) Application number: 19169203.7
(22) Date of filing: 15.04.2019
(51) Int. Cl.: G16H 50/20, G16H 50/50

(54) **NON-VERBAL COMMUNICATION**

(71) Applicant: Nokia Technologies Oy, 02610 Espoo (FI)
(72) Inventor: MARASCU, Alice, Dublin, 1 (IE); SALA, Alessandra, Dublin, 15 (IE); LINDHOLM, Harri, 00320 Helsinki (FI)
(74) Representative: Whiting, Gary

(57) **Abstract**

An apparatus, method and computer program is described comprising: receiving data relating to a first user from one or more sensors; processing the received data (by determining a symptomatic experience of the first user and determining one or more signals for inducing a trigger for a second user corresponding to the determined symptomatic experience); and communicating the one or more signals to the second user

## Description

### Field

The present specification relates to non-verbal communication between users, such as communications relating to symptomatic experience.

### Background

Arrangements for verbal communication between humans are known. There remains a need for further improvements related to non-verbal communication between users.

### Summary

In a first aspect, this specification describes an apparatus comprising: means for receiving data relating to a first user from one or more sensors; means for processing the received data, wherein the means for processing comprises: means for using a first processing model for determining, based on the received data, a symptomatic experience of the first user; and means for using a second processing model for determining one or more signals for inducing a trigger for a second user, wherein the determined one or more signals correspond to the determined symptomatic experience; and means for communicating the one or more signals to the second user.

The received data relating to the first user may comprise at least one of psychological data, biochemical data, behavioural data, physiological data, morphological data, biomechanical data, or social data.

Some embodiments further comprise means for inducing the trigger for the second user, wherein the means for inducing the trigger comprises means for causing at least one of: one or more visual, audible, haptic, environmental, olfactory, mental, emotional, or cognitive changes at the second user. The means for inducing the trigger for the second user may cause the second user to have a symptomatic experience corresponding, at least in part, to the determined symptomatic experience of the first user.

Some embodiments further comprise means for training at least one of the first processing model or the second processing model using generic data and corresponding symptomatic experience data from a plurality of users.

Some embodiments further comprise at least one of: means for training the first processing model using first personal data and corresponding symptomatic experience data from the first user; or means for training the second processing model using second personal data and corresponding symptomatic experience data from the second user.

At least one of the first processing model or the second processing model may be a machine learning model. Moreover, some embodiments further comprise means for performing a machine learning training phase, wherein one or more determined symptomatic experiences are labelled, during or after exposure of the first user to one or more stimuli, and the one or more labelled symptomatic experiences form a data set usable by the machine learning model. The machine learning model may comprise an unsupervised data mining algorithm to detect two or more consecutive determined symptomatic experiences of the first user.

The means for using the second processing model for determining one or more signals for inducing the trigger may comprise a mapping function for transforming the determined symptomatic experience of the first user into the one or more signals.

In some embodiments, the first user and the second user are the same user.

In a second aspect, this specification describes an apparatus comprising: means for receiving one or more signals for inducing a trigger for a second user corresponding to a determined symptomatic experience of a first user; and means for inducing the trigger for the second user using one or more trigger devices. The means for inducing the trigger for the second user comprises one or more of: means for inducing one or more haptic triggers; means for inducing one or more audio-visual triggers; means for inducing one or more environmental triggers; means for inducing one or more olfactory triggers; means for inducing one or more mental triggers; means for inducing one or more emotional triggers; or means for inducing one or more cognitive triggers.

In some embodiments, the first user and the second user are the same user.

In a third aspect, this specification describes a method comprising: receiving data relating to a first user from one or more sensors; processing the received data, wherein the means for processing comprises: using a first processing model for determining, based on the received data, a symptomatic experience of the first user; and using a second processing model for determining one or more signals for inducing a trigger for a second user, wherein the determined one or more signals correspond to the determined symptomatic experience; and communicating the one or more signals to the second user.

The received data relating to the first user may comprise at least one of psychological data, biochemical data, behavioural data, physiological data, morphological data, biomechanical data, or social data.

Some embodiments further comprise inducing the trigger for the second user, wherein inducing the trigger comprises causing at least one of: one or more visual, audible, haptic, environmental, olfactory, mental, emotional, or cognitive changes at the second user. Inducing the trigger for the second user may cause the second user to have a symptomatic experience corresponding, at least in part, to the determined symptomatic experience of the first user.

Some embodiments further comprise training at least one of the first processing model or the second processing model using generic data and corresponding symptomatic experience data from a plurality of users.

Some embodiments further comprise at least one of: training the first processing model using first personal data and corresponding symptomatic experience data from the first user; or training the second processing model using second personal data and corresponding symptomatic experience data from the second user.

Some embodiments further comprise performing a machine learning training phase, wherein one or more determined symptomatic experiences are labelled, during or after exposure of the first user to one or more stimuli, and the one or more labelled symptomatic experiences form a data set usable by the machine learning model. The machine learning model may comprise an unsupervised data mining algorithm to detect two or more consecutive determined symptomatic experiences of the first user.

The means for using the second processing model for determining one or more signals for inducing the trigger may comprise a mapping function for transforming the determined symptomatic experience of the first user into the one or more signals.

In some embodiments, the first user and the second user are the same user.

In a fourth aspect, this specification describes a method comprising: receiving one or more signals for inducing a trigger for a second user corresponding to a determined symptomatic experience of a first user; and inducing the trigger for the second user using one or more trigger devices.

In a fifth aspect, this specification describes an apparatus configured to perform any method as described with reference to the third or fourth aspects.

In a sixth aspect, this specification describes computer-readable instructions which, when executed by computing apparatus, cause the computing apparatus to perform any method as described with reference to the third or fourth aspects.

In a seventh aspect, this specification describes a computer program comprising instructions for causing an apparatus to perform at least the following: receiving data relating to a first user from one or more sensors; processing the received data, wherein the means for processing comprises: using a first processing model for determining, based on the received data, a symptomatic experience of the first user; and using a second processing model for determining one or more signals for inducing a trigger for a second user, wherein the determined one or more signals correspond to the determined symptomatic experience; and communicating the one or more signals to the second user. Moreover, this specification describes a computer program comprising instructions for causing an apparatus to perform at least the following: receiving one or more signals for inducing a trigger for a second user corresponding to a determined symptomatic experience of a first user; and inducing the trigger for the second user using one or more trigger devices.

In an eighth aspect, this specification describes a computer-readable medium (such as a non-transitory computer-readable medium) comprising program instructions stored thereon for performing at least the following: receiving data relating to a first user from one or more sensors; processing the received data, wherein the means for processing comprises: using a first processing model for determining, based on the received data, a symptomatic experience of the first user; and using a second processing model for determining one or more signals for inducing a trigger for a second user, wherein the determined one or more signals correspond to the determined symptomatic experience; and communicating the one or more signals to the second user. Moreover, this specification describes: a computer-readable medium (such as a non-transitory computer-readable medium) comprising program instructions stored thereon for performing at least the following: receiving one or more signals for inducing a trigger for a second user corresponding to a determined symptomatic experience of a first user; and inducing the trigger for the second user using one or more trigger devices.

In a ninth aspect, this specification describes an apparatus comprising: at least one processor; and at least one memory including computer program code which, when executed by the at least one processor, causes the apparatus to: receive data relating to a first user from one or more sensors; process the received data, wherein the means for processing comprises: using a first processing model for determining, based on the received data, a symptomatic experience of the first user; and using a second processing model for determining one or more signals for inducing a trigger for a second user, wherein the determined one or more signals correspond to the determined symptomatic experience; and communicate the one or more signals to the second user. Moreover, this specification describes an apparatus comprising: at least one processor; and at least one memory including computer program code which, when executed by the at least one processor, causes the apparatus to: receive one or more signals for inducing a trigger for a second user corresponding to a determined symptomatic experience of a first user; and induce the trigger for the second user using one or more trigger devices.

In a tenth aspect, this specification describes an apparatus comprising: a processing module for processing the received data, wherein the first processing module is configured to: use a first processing model for determining, based on the received data, a symptomatic experience of the first user; and use a second processing model for determining one or more signals for inducing a trigger for a second user, wherein the determined one or more signals correspond to the determined symptomatic experience; and a communication module for communicating the one or more signals to the second user. Moreover, this specification describes an apparatus comprising: a first module for receiving one or more signals for inducing a trigger for a second user corresponding to a determined symptomatic experience of a first user; and a second module for inducing the trigger for the second user using one or more trigger devices.

### Brief description of the drawings

Example embodiments will now be described, by way of non-limiting examples, with reference to the following schematic drawings, in which:
FIG. 1 is a block diagram of an example system;
FIG. 2 is a block diagram of a system in accordance with an example embodiment;
FIG. 3 is a flow chart of an algorithm in accordance with an example embodiment;
FIG. 4 is a flow chart of an algorithm in accordance with an example embodiment;
FIGS. 5 to 8, 9A and 9B are block diagrams of systems in accordance with example embodiments;
FIGS. 10A and 10B show processing models in accordance with example embodiments;
FIGS. 11 and 12 are flow charts of algorithms in accordance with example embodiments;
FIG. 13 is a block diagram of a system in accordance with an example embodiment; and
FIGS. 14A and 14B show tangible media, respectively a removable non-volatile memory unit and a compact disc (CD) storing computer-readable code which when run by a computer perform operations according to embodiments.

### Detailed description

In the description and drawings, like reference numerals refer to like elements throughout.

Many technologies have been developed to aid human communication. These include the formation of language, the development of telephony and modern virtual reality communication. Humans communicate both verbally (e.g. using written or oral language) and non-verbally (e.g. by gesture, body posture, mimicking, emotion etc.). Indeed, some experts argue that the majority of communication and interaction between humans occurs at a non-verbal level.

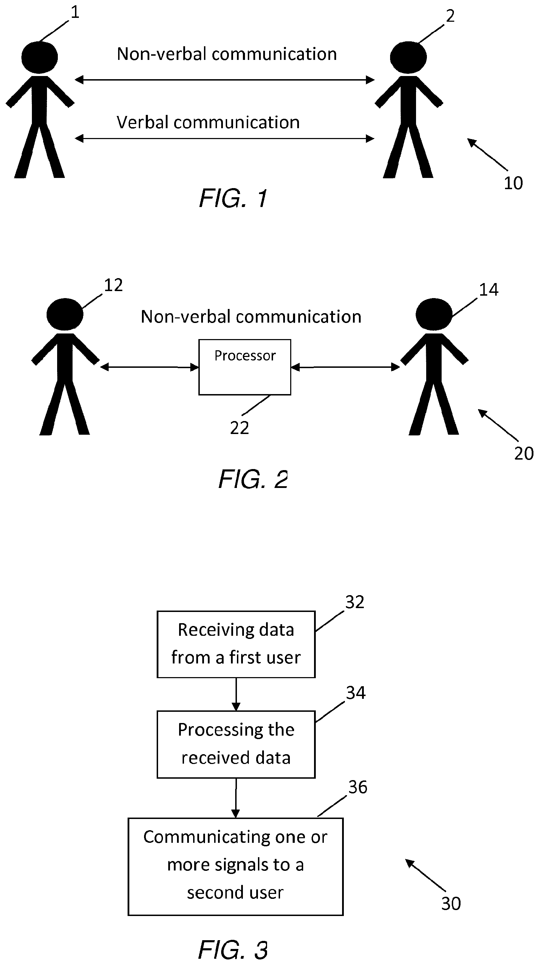

FIG. 1 is a block diagram of an example system, indicated generally by the reference numeral 10. The system 10 comprises a first person 1 and a second person 2 communicating through verbal communication and non-verbal communication. In some example embodiments, the first person 1 and the second person 2 may be separated by a considerable distance. Although there are many known technologies that allow effective verbal communication (captured clearly with verbal language) between the first person 1 and the second person 2 (even if separated by a considerable distance), there are fewer technologies to support non-verbal communication.

FIG. 2 is a block diagram of a system, indicated generally by the reference numeral 20, in accordance with an example embodiment. System 20 comprises a first user 12, a second user 14 and a processor 22. The first user 12 may communicate with the second user 14 through processor 22 in order to convey one or more symptomatic experiences in non-verbal communication. The processor 22 is described in further detail below.

Symptomatic experiences may include one or more of the experiences of feeling happy, sad, fearful, angry, surprised, physical pain, cold, hot, positivity, relaxation, engagement, dizziness, extraversion, awakeness, socialness, fitness, empathy, or any other experience that a user may feel and may be required to be communicated non-verbally.

In the use of the system 20, a symptomatic experience of the first user 12 may be perceived by the second user 14. For example, the second user 14 may experience a similar symptomatic experience to the first user 12 and/or may be caused to understand the symptomatic experience of the first user 12 in some way. Alternatively, or in addition, a symptomatic experience of the second user 14 may be perceived by the first user 12.

The system 20 may be used in various example scenarios that may require and may be improved by non-verbal communication. Such example scenarios may comprise, but are not limited to, medical scenarios, sports scenarios, and entertainment scenarios.

For example, in a medical scenario, a patient (such as the first user 12) may not be able to explain to a doctor (such as the second user 14) about any pain or discomfort he/she is feeling. As such, the processor 22 may be used for determining a symptomatic experience of the patient, and conveying the symptomatic experience to the doctor. The doctor may be provided with information about the symptomatic experience of the patient, such as 'dizzy', 'physical pain', 'cold', 'hot', and/or any other symptomatic experience. The doctor may also be provided with information about which body part the patient is feeling the symptomatic experience at.

In another example, in an entertainment scenario, a viewer of a movie or a player of a game may not completely understand how a character (such as the first user 12) of the movie or the game is feeling. The movie or gaming experience may be enhanced for the viewer or player (such as the second user 14) by non-verbal communication through the processor 22. A symptomatic experience of the character (such as happy, sad, fearful, angry, etc.) may be perceived by the viewer or player, such that the viewer or player may also feel a similar symptomatic experience as the symptomatic experience of the character.

In another example, in a sports scenario, a training player (such as a second user 14) who is just practising for a game may not be able to perceive the symptomatic experience that a player may feel in a real game. As such, the processor 22 may be used for determining one or more symptomatic experiences of a player in a real game (such as anxiety, or fearfulness), and conveying the symptomatic experiences to the training player. The determining of the symptomatic experiences and conveying of the symptomatic experiences may be performed at separate time instances, or may be performed at the same time instances in real time. The training player may have a better practice game (closely simulating a real game) if he/she feels the same anxiety that a player would feel in a real game, and may improve his/her playing skills.

In yet another sports scenario example, a viewer of a sports event (such as the second user 14) may have their experience or understanding enhanced by receiving non-verbal communication through the processor 22 regarding the symptomatic experience of a player in the sports event (such as the first user 12). Thus, a symptomatic experience of the player in the sports event (such as excitement, anger, anxiety, or fearfulness etc.) may be perceived by the viewer.

FIG. 3 is a flowchart of an algorithm, indicated generally by the reference numeral 30, in accordance with an example algorithm. At operation 32, data is received (for example at the processor 22) from a first user, such as the first user 12. At operation 34, the received data is processed (for example at the processor 22). At operation 36, one or more signals are communicated to a second user, such as the second user 14. The one or more signals are determined based, at least in part, on the data received from the first user 12.

In an example embodiment, the received data of the first user 12 may comprise one or more of psychological data, biochemical data, behavioural data, physiological data, morphological data, biomechanical data, and social data. As described further below, the data may be collected from one or more sensors at the first user 12. The received data may be used for determining one or more symptomatic experiences of the first user 12 using cognitive analytics. In order to determine symptomatic experience(s) corresponding to a set of received data, a processing model may be used, where the processing model is trained with data from one or a plurality of users.

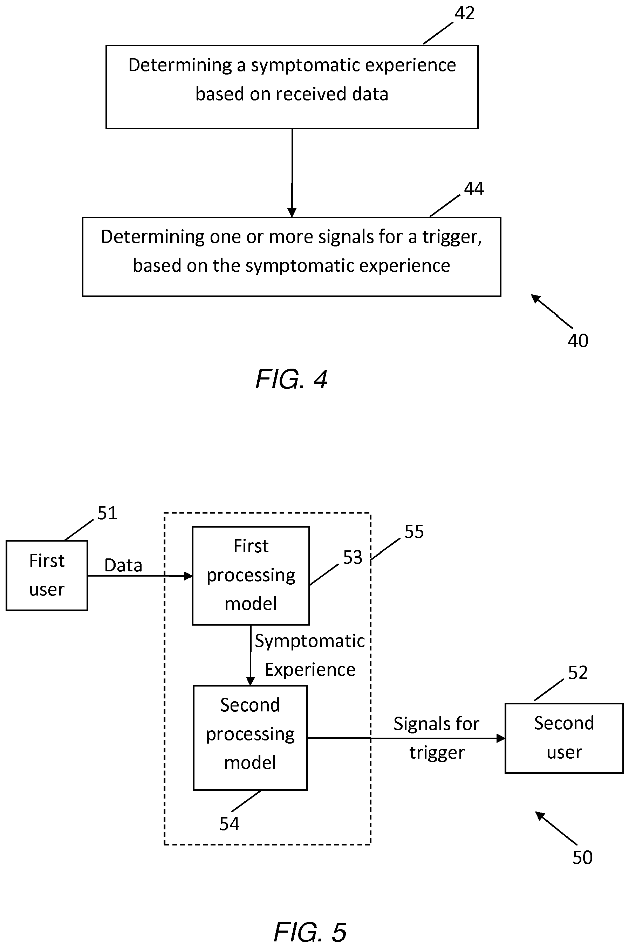

FIG. 4 is a flowchart of an algorithm, indicated generally by a reference numeral 40, in accordance with an example embodiment. The algorithm 40 is an example implementation of the operation 34 of the algorithm 30 described above.

The algorithm 40 starts at operation 42, where a symptomatic experience of the first user 12 is determined based on the received data. At operation 44, one or more signals are determined for inducing a trigger for the second user 14. The one or more signals may correspond to the determined symptomatic experience of the first user 12. In an example embodiment, inducing said trigger may allow the second user 14 to have a similar symptomatic experience as the first user 12. For example, if it is determined that the symptomatic experience of the first user 12 is 'happy', the one or more signals are determined so that the trigger induced allows the second user 14 to feel 'happy'. For example, showing photos of small puppies may make the second user 14 happy, and therefore the one or more signals are determined to be photos of small puppies.

In an example embodiment, the symptomatic experience is determined in operation 42 using a first processing model. In an example embodiment, the one or more signals for inducing the trigger are determined in the operation 44 using a second processing model. Example first and second processing models are described further below.

FIG. 5 is a block diagram of a system, indicated generally by the reference numeral 50, in accordance with an example embodiment. The system 50 comprises a first user 51 (similar to the first user 12), a second user 52 (similar to the second user 14), a first processing model 53, and a second processing model 54. The first processing model 53 and the second processing model 54 may optionally be comprised within processor 55 (similar to processor 22). Data may be received (operation 32) from the first user 51 and the first processing model 53 may be used for determining a symptomatic experience (operation 42) of the first user 51 based on the received data. The symptomatic experience is provided to the second processing model 54 and the second processing model 54 may be used for determining (operation 44) one or more signals for inducing a trigger for the second user 52, which signals are communicated (operation 36) to the second user 52. The determined one or more signals may correspond to the symptomatic experience.

In an example embodiment, the second processing model 54 may use a mapping function for transforming the determined symptomatic experience of the first user 51 into the one or more signals for inducing a trigger for the second user 52. The mapping of the second processing model 54 may be trained using machine learning principles, as described further below.

The first processing model 53 and the second processing model 54 may be used in real time for conveying a symptomatic experience of the first user 51 to the second user 52 using one or more triggers. This may be done when the first processing model 53 and the second processing model 54 are already trained with generic data of a plurality of users and/or personal data of the first user 51 and/or the second user 52. Data may be collected with a continuous monitoring and processed according to the first processing model 53, where the symptomatic experience is determined based on the received data. The symptomatic experience may be determined by matching patterns of the received data against a database (e.g. trained model) of patterns for symptomatic experiences, using, for example, data mining analytics. When the determined symptomatic experience data is provided to the second processing model 54, a mapping function is used for performing transformation and determining one or more signals that may be used for inducing a trigger for the second user 52.

For example, the mapping function may be used for transformation of one or more symptomatic experiences of the first user 51 into one or more signals for inducing one or more triggers for the second user 52, such that the one or more induced triggers allow the second user 52 to experience one or more symptomatic experiences similar to that of the first user 51. Example triggers and trigger devices are discussed further below.

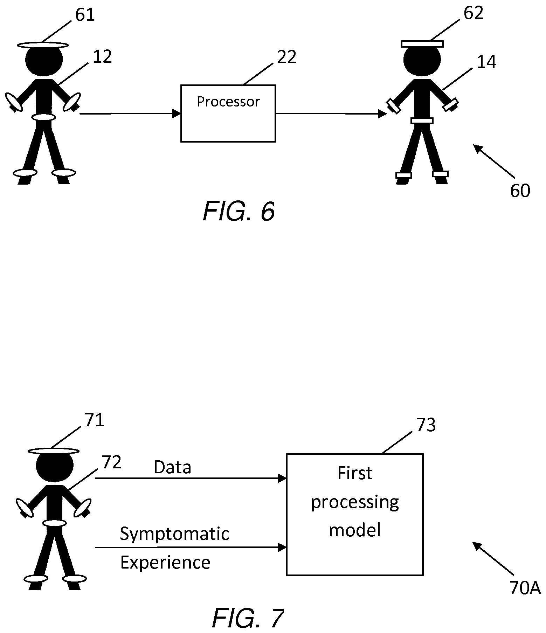

FIG. 6 is a block diagram of a system, indicated generally by the reference numeral 60, in accordance with an example embodiment. The system 60 comprises the first user 12, the processor 22, and the second user 14 described above. One or more sensors 61 (illustrated by ovals on the head, wrists, abdomen, and ankles of the first user 12) may be used for collecting data related to the first user 12. For example, one or more of the one or more sensors 61 may be comprised within one or more wearable devices worn by the first user 12. One or more trigger devices 62 (illustrated by rectangles on the head, wrists, abdomen, and ankles of the first user 12) may be used for inducing a trigger for the second user 14. For example, one or more of the trigger device(s) 62 may be comprised within one or more wearable or non-wearable devices used by the second user 14.

It is not essential that the sensors 61 and the trigger devices 62 are wearable by the first user 12 and the second device 14. For example, one or more of the sensors 61 may be a camera for capturing facial expressions of the first user 12; and/or the one or more of the trigger devices 62 may be an external screen for inducing a trigger by showing images or videos to the second user 14.

The system 60 is described with sensors 61 at the first user 12 and trigger devices 62 at the second user to enable non-verbal communication from the first user to the second user. Of course, such communication could be provided from the second user to the first user, or could be provided in both directions. Thus, a system can be provided to enable both verbal and non-verbal communication between two users, in both directions.

In an example embodiment, the processor 22 may comprise means for storing streaming data received from the first user 12 and/or the second user 14, means for analysing the streaming data, and communication means for communicating with the first user 12 and second user 14 (such as wireless or wired communications).

It would be appreciated that the positions of the plurality of sensors 61 and trigger devices 62 are example positions. One or more of the sensors 61 may have different positions, and/or one or more of the trigger devices 62 may have different positions.

In an example embodiment, the one or more sensors 61 comprise one or more of: visual sensor(s), auditory sensor(s), infrared sensor(s), heart rate monitor(s), accelerometer(s), electrodermal sensor(s), electroencephalogram (EEG) sensor(s), optical coherence tomography (OCT) sensor(s), electrocardiography (ECG) sensor(s), photoplethysmography (PPG) sensor(s), or breath sensor(s).

As noted above, a variety of sensor types may be used for sensing aspects of the first user 12 (and/or the second user 14). Visual sensors (if provided) may include a camera, such that visual indicators of the first user 12 may be recorded. The visual indicators may include, for example, one or more of facial expressions, body movements or body positions. Auditory sensor(s) (if provided) may include sound recorders, such that auditory indicators of the first user 12 may be recorded. The auditory indicators may include, for example, changes in the voice or tone of the first user 12. Infrared sensors may also be used for determining facial emotional patters, finger temperature, skin temperature, and the like. Electrodermal sensor(s) may be used for determining electrical variations of the skin. Electrocardiography (ECG) sensor(s) may be used for determining variations in heart rate of the first user 12. One or more sensors may be used for collecting data for analysing emotional biomarkers in human biogases and biofluids (such as breath, saliva, sweat, etc.). The skilled person will be aware of many other sensor types that could be used.

In an example embodiment, physiological data may include physical measurements (such as variations in heart rate); emotional data may include facial expression patterns, gestures, postures, voice, etc. (for example, from a video capture); and biological data may include, for example, a saliva composition.

In an example embodiment, the one or more trigger devices 62 are configured to induce, for the second user 14, one or more of the following triggers: visual triggers, audible (auditory or hearing) triggers, haptic (tactile or touch) triggers, environmental triggers, olfactory (smell) triggers, gustatory (taste) triggers, mental triggers, emotional triggers, or cognitive triggers. The skilled person will be aware of many other triggers that could be induced.

The triggers are induced such that one or more of the human senses are triggered, and in turn, the second user 14 may feel one or more symptomatic experiences. For example, visual triggers may include display of photos, videos, texts, etc., to be seen by the second user 14; audible triggers may include playing of sounds to be heard by the second user 14; haptic triggers may include vibrations or pressure to be felt by the second user 14; olfactory triggers may include providing smells to be smelt by the second user 14; and environmental triggers may include anything (such as a temperature change) to be felt, smelt, heard, or seen by the second user 14.

In an example embodiment, if the symptomatic experience of the first user 12 is related to physical pain or discomfort, it may be preferred to induce a trigger for the second user 14 such that the second user 14 only feels unpleasant emotionally, but not physically (as physically hurting the second user 14 may not be an ethical practice). For example, the trigger may include a video related to a death, so that the second user 14 may feel unpleasant emotionally, but may not feel any physical pain. In another example, if the symptomatic experience of the first user 12 is related to physical pain or discomfort, the second user 14 is informed (for example, by means of a notification) that the first user 12 is feeling physical pain, and may also be informed of the body part where the first user 12 may be feeling the physical pain or discomfort. As such, the trigger may only comprise a notification, and may not cause the second user 14 to have a similar symptomatic experience as the first user 12. This may be beneficial in medical scenarios, such that a doctor (e.g. the second user 14) may be able to understand how a patient (e.g. the first user 12) is feeling.

In an example embodiment, the first user 12 may control which symptomatic experiences may or may not be conveyed to the second user 14. For example, the first user 12 may configure the processor 22 such that any symptomatic experience other than 'angry' may be conveyed to the second user 14. Therefore, when a symptomatic experience of the first user 12 is determined (operation 42) to be 'happy', 'sad', 'surprised', etc., one or more signals are determined (operation 44) for inducing a trigger for the second user 14; and when a symptomatic experience of the first user 12 is determined (operation 42) to be 'angry', the symptomatic experience may not be provided to the second processing model 54, the one or more signals are not determined, and no triggers are induced for the second user 14. Alternatively, or in addition, some triggers may be induced for the second user, but the triggers may not relate to the symptomatic experience of 'angry', and may relate to any other symptomatic experience chosen by the first user 12. For example, when the first user 12 is feeling angry, the first user 12 may choose a symptomatic experience 'sad' for the second user 14, such that triggers related to the symptomatic experience 'sad' are induced for the second user 14.

In another example embodiment, the second user 14 may control which symptomatic experiences may or may not be conveyed to the second user 14. For example, the second user 14 may configure the processor 22 and/or one or more trigger devices to not induce triggers related to the symptomatic experience 'angry'. As discussed above, when the first user 12 is feeling angry, no triggers or any triggers other than triggers related to the symptomatic experience 'angry' are induced for the second user 14.

FIG. 7 is a block diagram of a system, indicated generally by the reference numeral 70A, in accordance with an example embodiment. System 70A comprises user(s) 72, sensors 71 and a first processing model 73.

In an example embodiment, the first processing model 73 is trained with generic data and corresponding symptomatic experience data from a plurality of users 72. Generic data may be obtained from one or more of the sensors 71 used by one or more of the plurality of users 72. The first processing model 73 may be trained with generic data of the plurality of users 72 and a known symptomatic experience data corresponding to the generic data. For example, when a user 72 is happy, the user 72 may provide the symptomatic experience data that the user 72 is 'happy' to the first processing model 73 (e.g. in the form of a data label), and the sensors 71 may provide data (such as psychological data, biochemical data, behavioural data, physiological data, morphological data, biomechanical data and/or social data) related to the symptomatic experience of being happy. The plurality of users 72 may be monitored over a time period, such that a larger set of symptomatic experience data and corresponding generic data are captured. Such an arrangement may enable the first processing model 73 to be trained (e.g. using the principles of machine learning).

It should be appreciated that different users may show different symptoms while having the same symptomatic experience. For example, a heart rate of one user while being happy may be different from the heart rate of another user while being happy. Therefore, training the first processing model 73 with data from a plurality of users allows creating a large dataset, and possibly learning various ranges of data that may be corresponding to a particular symptomatic experience.

In another example embodiment, alternatively or in addition to training the first processing model 73 with generic data, the first processing model 73 is trained with first personal data and corresponding symptomatic experience data from a first user. For example, the user 72 is the first user (similar to the first user 12). The first user may be monitored over an extended period of time, for example a number hours, days, months or years, such that a large amount of data (i.e. sensor data), for example in the order of megabytes, gigabytes, terabytes or more, is received from the first user.

The first processing model 73 may comprise stored datasets with the received data and corresponding symptomatic experience. For example, each symptomatic experience dataset may comprise a label (such as 'happy', 'sad', etc.), and a pattern of streaming time series data. The time series data may be represented in a simplified format, such as a multidimensional point representation, or a graph. The labels for symptomatic experience may be learnt from the training inputs for the symptomatic experience data from the plurality of users 72. In one example, the data and the symptomatic experience data may be collected from one or more users under medical supervision.

In an example embodiment, the stored datasets may comprise bodily maps, and may comprise a labelled dataset. The labels in the labelled dataset may comprise medical labels (such as medical symptoms obtained from known medical sources), emotional labels (such as 'happy', 'sad', etc.), and images, sounds, colours, or any other triggers labelled with known corresponding symptomatic experiences.

The trained first processing model 73 may be used as the first processing model 53 for determining (operation 42) symptomatic experience based on the received data in an implementation of the algorithm 40 described above.

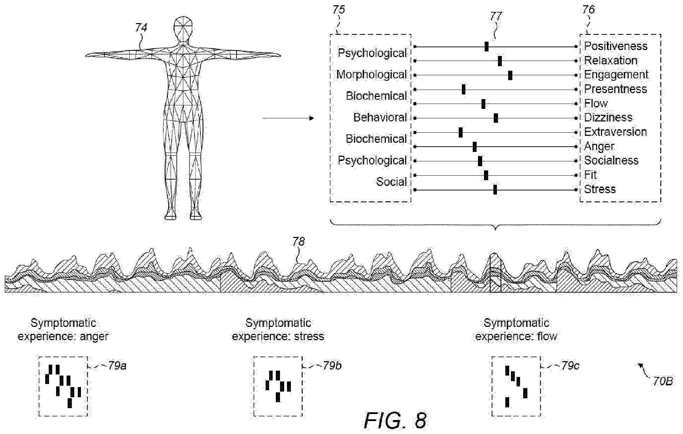

FIG. 8 is a block diagram of a system, indicated generally by the reference numeral 70B, in accordance with an example embodiment. System 70B provides an example of how a symptomatic experience dataset may be obtained. As discussed above, the symptomatic experience dataset may be comprised within the first processing model 73 as the first processing model 73 is trained with data and symptomatic experience of one or more users. System 70B comprises a user 74, user data 75, labels 76, streaming time series data representation 77, streaming time series data graph 78, and one or more patterns of streaming time series data 79a, 79b, and 79c. As shown in system 70B, a plurality of user data 75 is collected from the user 74 (for example from sensors associated with the user 74). The user data 75 may comprise one or more of psychological, biochemical, behavioural, physiological, morphological data, biomechanical data and/or social data. The labels 76 may comprise a plurality of symptomatic experience labels. Values of the user data 75 corresponding to each of the labels 76 may be determined and represented in a streaming time series data. The streaming time series data may be represented as a multi-dimensional point representation, such as streaming time series data representation 77. Alternatively, or in addition, the streaming time series data may be represented as a graph, such as streaming time series data graph 78. Patterns 79 may show a representation of time series data, for example for a respective symptomatic experience label. For example, pattern 79a shows a pattern for user data corresponding to the symptomatic experience label 'anger'; pattern 79b shows a pattern for user data corresponding to the symptomatic experience label 'stress'; and pattern 79c shows a pattern for user data corresponding to the symptomatic experience label 'flow'.

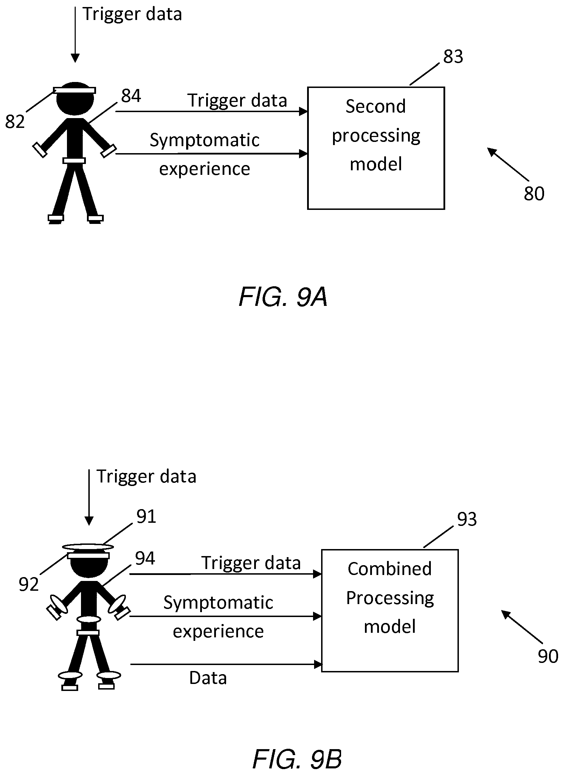

FIG. 9A is a block diagram of a system, indicated generally by the reference numeral 80, in accordance with an example embodiment. System 80 comprises user(s) 84, second processing model 83, and trigger devices 82.

In an example embodiment, the second processing model 83 is trained with generic trigger data and corresponding symptomatic experience data from a plurality of users 84. The generic trigger data is used for inducing triggers for the user(s) 84, for example, using the trigger devices 82. The generic trigger data with which a trigger is induced at the user(s) 84 is also provided to the second processing model 83, together with corresponding symptomatic experience data. For example, the generic trigger data comprises a photo of small puppies, which may make the user(s) 84 happy. Thus, the trigger data (of the photo of small puppies) and a symptomatic experience data of 'happy' are provided for training the second processing model 83. Such an arrangement may enable the second processing model 83 to be trained (e.g. using the principles of machine learning).

In an example embodiment, alternatively or in addition to training the second processing model 83 with trigger data and symptomatic experience data of a plurality of users, the second processing model 83 is trained using a second personal data and corresponding symptomatic experience data from a second user. For example, the user 84 may be the second user (similar to the second user 14). The trigger data therefore may comprise specific trigger data that causes the second user to have a specific symptomatic experience. For example, the photo of small puppies may usually make people happy, except for a person who is scared of dogs. If the second user does not feel happy looking at a photo of puppies, the second user may provide any trigger data (for example a favourite song that makes the second user happy) and a corresponding 'happy' symptomatic experience data to the second processing model 83. As such, the second processing model 83 may be trained with specific information of the second user, including what kinds of triggers may be required to make the second user have a particular symptomatic experience.

In an example embodiment, the trained second processing model 83 is used as the second processing model 54 for determining (operation 44) one or more signals for inducing a trigger for the second user based on the symptomatic experience.

In an example embodiment, the first user and the second user are the same user, such as a person A. For example, in system 70A, the person A is the user 72, and the first processing model 73 is trained with first personal data and corresponding symptomatic experiences of the person A (i.e. user 72). In system 80, the person A is the user 84, and the second processing model 73 is trained with second personal data and corresponding symptomatic experiences of the person A (i.e. user 84). The first personal data may comprise data (such as psychological data, biochemical data, behavioural data, physiological data, morphological data, biomechanical data and/or social data) collected from the sensors 71 worn by the person A, and the second personal data may comprise trigger data that causes the person A to have a specific symptomatic experience. Viewing in conjunction with FIG. 5, the first processing model 53 (such as the first processing model 73) is trained with first personal data of the person A, and the second processing model 54 (such as the second processing model 83) is trained with the second personal data of the person A. In one example, a first symptomatic experience of person A can be determined (operation 42) at a first time instance, and signals may be determined (operation 44) for inducing one or more triggers for the same person A to experience the first symptomatic experience at a second time instance. Therefore, the person A is the first user 51 at the first time instance, and the same person A is the second user 52 at the second time instance.

FIG. 9B is a block diagram of a system, indicated generally by the reference numeral 90, in accordance with an example embodiment. The system 90 comprises a combined processing model 93, a user 94, sensor(s) 91, and trigger device(s) 92. The combined processing model 93 may incorporate both the first processing model 73 and the second processing model 83 described above. Indeed, the combined processing model 93 may comprise a combination of those models, for example, incorporating a dataset of a plurality of symptomatic experiences (such as happy, sad, etc.), corresponding trigger data that allows the user 94 to experience the plurality of symptomatic experiences, and corresponding sensor data (such as psychological data, biochemical data, behavioural data, physiological data, morphological data, biomechanical data, and/or social data) of the user 94 while the user feels each of the symptomatic experiences.

In one example embodiment, at least one of the first processing model 73, the second processing model 83, and the combined processing model 93 are machine learning models (for example neural networks).

One or more of the first processing model 53, 73, the second processing model 54, 83 and the combined processing model 93 may be implemented as neural networks or other machine-learning algorithms.

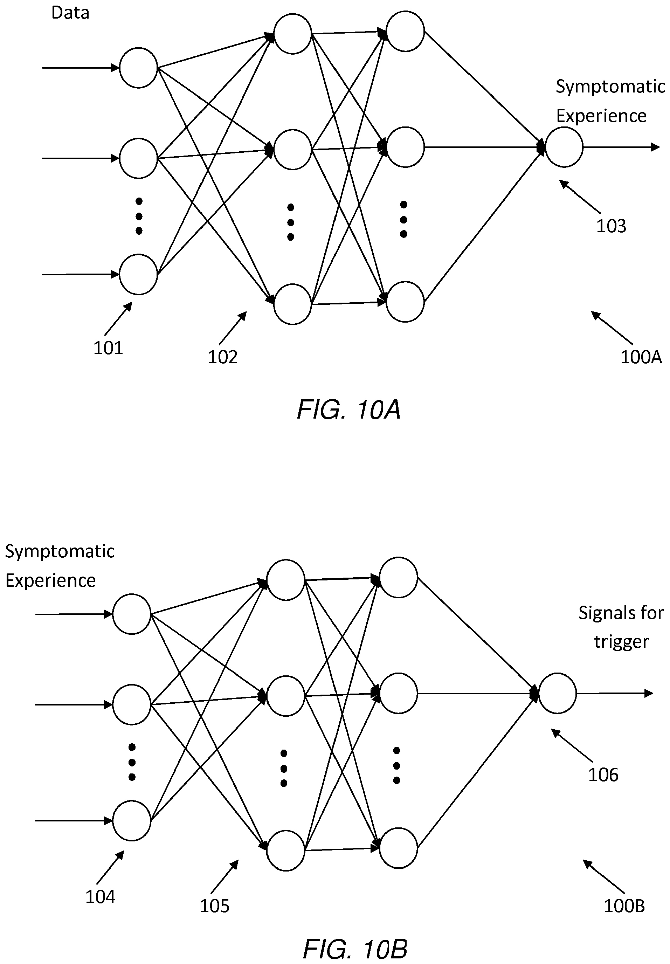

FIGS. 10A and 10B show first and second neural networks, indicated generally by the reference numerals 100A and 100B respectively, used in some example embodiments. For example, the first processing model 53 or 73 may comprise the neural network 100A. The neural network 100A may be trained, as described above with reference to FIG. 7. The neural network 100A comprises an input layer 101, one or more hidden layers 102, and an output layer 103. At the input layer 101, the data relating to the first user is received as an input. The hidden layers 102 may comprise a plurality of hidden nodes, where the cognitive analytics are performed corresponding to the data received. At the output layer 103, one or more symptomatic experiences corresponding to the input data are provided as an output.

The second processing model 54 or 83 may comprise the neural network 100B. The neural network 100B may be trained, as described above with reference to FIG. 9A. The neural network 100B comprises an input layer 104, one or more hidden layers 105, and an output layer 106. At the input layer 104, a symptomatic experience is received as an input. The hidden layers 105 may comprise a plurality of hidden nodes, where the transformation is performed using the mapping function. At the output layer 106, one or more signals for inducing a trigger (corresponding to the symptomatic experience) for the second user are provided as an output.

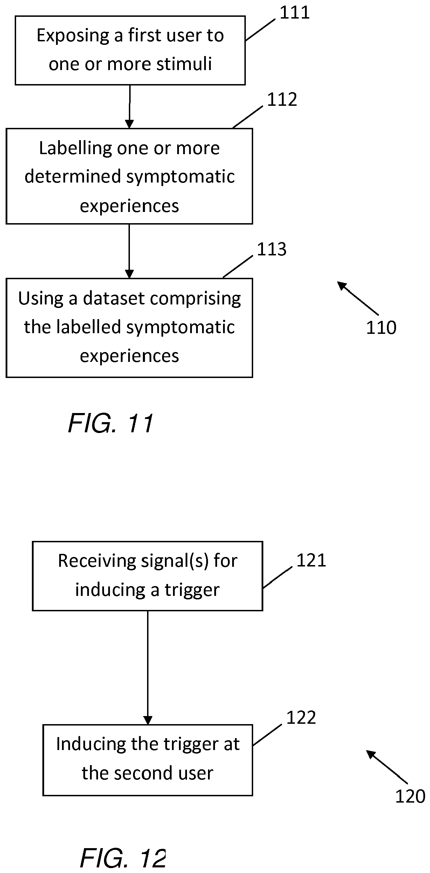

FIG. 11 is a flowchart of an algorithm, indicated generally by the reference numeral 110, in accordance with an example embodiment. The operations of algorithm 110 may be performed at a machine learning training phase, where a machine learning model(s) (such as the first processing model 53, 73, 110A, the second processing model 54, 83, 110B or the combined processing model 93) is/are trained before being used by the first user 12 and/or the second user 14. At operation 111, the first user 12 is exposed to one or more stimuli (such as triggers). At operation 112, one or more determined symptomatic experiences are labelled. The symptomatic experiences may be labelled during or after exposing the first user to the one or more stimuli. During the machine learning training phase, the labelling may be performed by the first user 12 or may be performed by an external user. The labelled symptomatic experiences may form a dataset, where the dataset may be used, at operation 113, by the machine learning model. As such, the first user 12 may induce triggers for himself/herself (instead of inducing triggers for the second user 14), and may label symptomatic experiences felt by himself/herself (first user 12) while being induced with the triggers.

In an example embodiment, the machine learning model comprises an unsupervised data-mining algorithm. The unsupervised data-mining algorithm may receive, as an input, continuous data from the first user 12, and may therefore be able to detect two or more consecutive determined symptomatic experiences of the first user 12. As such, the unsupervised data mining algorithm may be used for detecting separating points between consecutive symptomatic experiences of the first user 12. In an example embodiment, past symptomatic experience data (historical data related to the past symptomatic experiences) of the first user 12 may be stored, and may be used by the machine learning model for determining the symptomatic experiences of the first user 12.

FIG. 12 is a flowchart of an algorithm, indicated generally by the reference numeral 120, in accordance with an example embodiment. At operation 121, one or more signals for inducing a trigger for a second user may be received. The one or more signals may be corresponding to a determined symptomatic experience of a first user. At operation 122, the trigger is induced for the second user using one or more trigger devices. The induced trigger may allow the second user to have a similar symptomatic experience as the first user.

In an example embodiment, the one or more signals may be used for inducing a plurality of triggers for the second user. The plurality of triggers may comprise one or more of visual, audible, haptic (tactile or touch), environmental, olfactory (smell), gustatory (taste), mental, emotional, or cognitive triggers.

In an example embodiment, an order of inducing (operation 122) the plurality of triggers is based on an order of an expected impact of the triggers on the brain of the second user. The order of the expected impact may be known from neuroscience results, and may be based on the speeds required for the triggers to reach the brain. For example, the order of inducing the triggers is in decreasing order of the expected impact on the brain. Therefore, in one example, the plurality of triggers may be induced in the following order: visual trigger(s), audible trigger(s), olfactory trigger(s), gustatory trigger(s), haptic triggers. The brain may assign higher importance to certain triggers compared to other triggers.

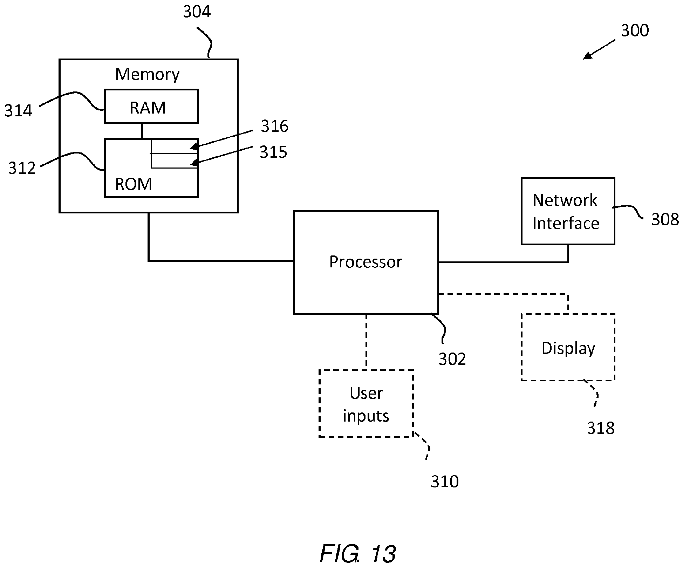

For completeness, FIG. 13 is a schematic diagram of components of one or more of the example embodiments described previously, which hereafter are referred to generically as a processing system 300. The processing system 300 may, for example, be the apparatus referred to in the claims below.

The processing system 300 may have a processor 302, a memory 304 coupled to the processor and comprised of a RAM 314 and a ROM 312, and, optionally, a user input 310 and a display 318. The processing system 300 may comprise one or more network/apparatus interfaces 308 for connection to a network/apparatus, e.g. a modem which may be wired or wireless. The interface 308 may also operate as a connection to other apparatus such as device/apparatus which is not network side apparatus. Thus, direct connection between devices/apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 304 stores, amongst other things, an operating system 315 and may store software applications 316. The RAM 314 of the memory 304 is used by the processor 302 for the temporary storage of data. The operating system 315 may contain code which, when executed by the processor implements aspects of the algorithms 30, 40, 110, and 120 described above. Note that in the case of small device/apparatus the memory can be most suitable for small size usage i.e. not always a hard disk drive (HDD) or a solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/apparatus such as IoT device/apparatus i.e. embedded to very small size

In some example embodiments, the processing system 300 may also be associated with external software applications. These may be applications stored on a remote server device/apparatus and may run partly or exclusively on the remote server device/apparatus. These applications may be termed cloud-hosted applications. The processing system 300 may be in communication with the remote server device/apparatus in order to utilize the software application stored there.

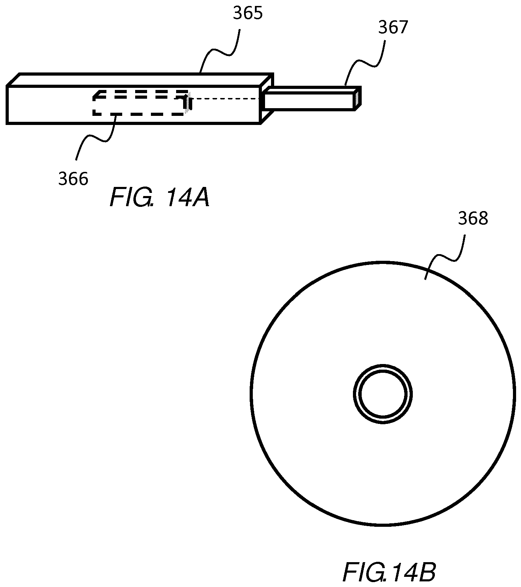

FIGs. 14A and 14B show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The internal memory 366 may be accessed by a computer system via a connector 367. The CD 368 may be a CD-ROM or a DVD or similar. Other forms of tangible storage media may be used. Tangible media can be any device/apparatus capable of storing data/information which data/information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices/apparatus and other devices/apparatus. References to computer program, instructions, code etc. should be understood to express software for a programmable processor firmware such as the programmable content of a hardware device/apparatus as instructions for a processor or configured or configuration settings for a fixed function device/apparatus, gate array, programmable logic device/apparatus, etc.

If desired, the different functions discussed herein may be performed in a different order and/or concurrently with each other. Furthermore, if desired, one or more of the above-described functions may be optional or may be combined. Similarly, it will also be appreciated that the flow diagram of Figures 3, 4, 11, 12 are examples only and that various operations depicted therein may be omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the above describes various examples, these descriptions should not be viewed in a limiting sense. Rather, there are several variations and modifications which may be made without departing from the scope of the present invention as defined in the appended claims.

## Claims

1. An apparatus comprising:
means for receiving data relating to a first user from one or more sensors;
means for processing the received data, wherein the means for processing comprises:
means for using a first processing model for determining, based on the received data, a symptomatic experience of the first user; and
means for using a second processing model for determining one or more signals for inducing a trigger for a second user, wherein the determined one or more signals correspond to the determined symptomatic experience; and means for communicating the one or more signals to the second user.

2. An apparatus as claimed in claim 1, wherein the received data relating to the first user comprises at least one of psychological data, biochemical data, behavioural data, physiological data, morphological data, biomechanical data, or social data.

3. An apparatus as claimed in claim 1 or claim 2, further comprising means for inducing the trigger for the second user, wherein the means for inducing the trigger comprises means for causing at least one of: one or more visual, audible, haptic, environmental, olfactory, mental, emotional, or cognitive changes at the second user.

4. An apparatus as claimed in claim 3, wherein the means for inducing the trigger for the second user causes the second user to have a symptomatic experience corresponding, at least in part, to the determined symptomatic experience of the first user.

5. An apparatus as claimed in any one of the preceding claims, further comprising means for training at least one of the first processing model or the second processing model using generic data and corresponding symptomatic experience data from a plurality of users.

6. An apparatus as claimed in any one of the preceding claims, further comprising at least one of:
means for training the first processing model using first personal data and corresponding symptomatic experience data from the first user; or
means for training the second processing model using second personal data and corresponding symptomatic experience data from the second user.

7. An apparatus as claimed in any one of the preceding claims, wherein at least one of the first processing model or the second processing model is a machine learning model.

8. An apparatus as claimed in claim 7, further comprising means for performing a machine learning training phase, wherein one or more determined symptomatic experiences are labelled, during or after exposure of the first user to one or more stimuli, and the one or more labelled symptomatic experiences form a data set usable by the machine learning model.

9. An apparatus as claimed in any one of claims 7 and 8, wherein the machine learning model comprises an unsupervised data mining algorithm to detect two or more consecutive determined symptomatic experiences of the first user.

10. An apparatus as claimed in any one of the preceding claims, wherein the means for using the second processing model for determining one or more signals for inducing the trigger comprises a mapping function for transforming the determined symptomatic experience of the first user into the one or more signals.

11. An apparatus comprising:
means for receiving one or more signals for inducing a trigger for a second user corresponding to a determined symptomatic experience of a first user; and
means for inducing the trigger for the second user using one or more trigger devices.

12. An apparatus as claimed in claim 11, wherein the means for inducing the trigger for the second user comprises one or more of:
means for inducing one or more haptic triggers;
means for inducing one or more audio-visual triggers;
means for inducing one or more environmental triggers;
means for inducing one or more olfactory triggers;
means for inducing one or more mental triggers;
means for inducing one or more emotional triggers; or
means for inducing one or more cognitive triggers.

13. An apparatus as claimed in any one of the preceding claims, wherein the first user and the second user are the same user.

14. A method comprising:
receiving data relating to a first user from one or more sensors;
processing the received data, wherein the means for processing comprises:
using a first processing model for determining, based on the received data, a symptomatic experience of the first user; and
using a second processing model for determining one or more signals for inducing a trigger for a second user, wherein the determined one or more signals correspond to the determined symptomatic experience; and communicating the one or more signals to the second user.

15. A computer program comprising instructions for causing an apparatus to perform at least the following:
receiving data relating to a first user from one or more sensors;
processing the received data, wherein the means for processing comprises:
using a first processing model for determining, based on the received data, a symptomatic experience of the first user; and
using a second processing model for determining one or more signals for inducing a trigger for a second user, wherein the determined one or more signals correspond to the determined symptomatic experience; and communicating the one or more signals to the second user.
